## Europäisches Patentamt

## European Patent Office

## Office européen des brevets

(19)

(11) Veröffentlichungsnummer: **0 236 971**
**B1**

(12) **EUROPÄISCHE PATENTSCHRIFT**

(45) Veröffentlichungstag der Patentschrift: **11.07.90**

(21) Anmeldenummer: **87103194.4**

(22) Anmeldetag: **07.03.87**

(51) Int. Cl.⁵: **C 07 D 487/22** //
(C07D487/22, 257:00, 209:00,
209:00, 209:00, 209:00)

(54) **Verfahren zum Isolieren und Reinigen von Hämin.**

(30) Priorität: **12.03.86 DE 3608091**

(43) Veröffentlichungstag der Anmeldung:
**16.09.87 Patentblatt 87/38**

(45) Bekanntmachung des Hinweises auf die
Patenterteilung:
**11.07.90 Patentblatt 90/28**

(84) Benannte Vertragsstaaten:
**AT BE CH DE ES FR GB IT LI NL**

(56) Entgegenhaltungen:
EP-A-0 013 055
EP-A-0 068 537
FR-A-2 274 230

(73) Patentinhaber: **BASF Aktiengesellschaft
Carl-Bosch-Strasse 38
D-6700 Ludwigshafen (DE)**

(72) Erfinder: **Schultze, Hans, Dr.
Werftweg 21
D-2082 Moorrege (DE)**

EP 0 236 971 B1

# Beschreibung

Die Erfindung betrifft ein Verfahren zum Isolieren und Reinigen von Hämin durch katalysierte Kristallisation aus wäßriger Lösung oder Suspension. Es gelingt normalerweise nicht, Hämin aus wäßriger Lösung zur Kristallisation zu bringen.

Nach den bisher bekannten Verfahren gelingt die Isolierung des Hämins aus Blut oder Hämoglobin nur bie Anwendung unverhältnismäßig großer Mengen von organischen Lüsungsmitteln. Für eine technische Produktion stellen diese Lösungsmittel den weitaus größten Kostenfaktor der und bringen darüber hinaus Umweltprobleme mit sich.

Bereits 1853 hat Teichmann die nach ihm benannten Kristalle des Hämins durch Einwirkung von Eisessig auf Blut in Gegewart von Salz erhalten. Diese mikroskopische Blutnachweismethode wurde später von Schalfejew zum Verfahren ausgearbeitet und wird noch heute als übliche Herstellmethode für Hämin beschrieben (Ullmann, Encyklopädie d. tech. Chemie, IV. Aufl., 1976, Bd. 11, S. 129). Hierbei werden zur Herstellung von 1 kg Hämin ca. 800 l Eisessig benötigt und zum Umkristallisieren zusätzlich 5 l Pyridin, 1 l Chloroform und wieder 70 l Eisessig. Die Reinhäminausbeute beträgt nur etwa 70% der theoretisch erreichbaren Menge. Rund 20% gehen allein beim Umkristallisieren verloren.

Andere bekannte Verfahren verwenden zur Hämin-Globin-Trennung von angesäuertem Hämoglobin Methanol (EP—B—68537), Ethanol, ggf. im Gemisch mit Glykol oder Glycerin (DE—A 2 526 596), Dimethylformamid, Methyläthylketon oder Aceton (Ztschr. f. physiol. Chem. Bd. 80, 1912, S. 35). Die benötigte Menge an den genannten organischen Lösungsmitteln beträgt 200 bis 500 l für 1 kg Hämin und ergibt dann nur ein stark verunreinigtes Hämin.

Nach dem US—Patent 4 330 463 wird Hämoglobin mit Salzsäure versetzt, sprühgetrocknet, und das so erhaltene saure Trockenblut mehrfach mit einer Ethanol-Methanol-Mischung extrahiert, wobei das Hämin herausgelöst wird. Hierzu werden über 1000 l Lösungsmittel pro kg Hämin gebraucht.

Der Erfindung lag die Aufgabe zugrunde, ein Verfahren zum Isolieren und Reinigen von Hämin zu schaffen, das ohne Verwendung von organischen Lösungsmitteln durchgeführt werden kann, und bei dem möglichst reines Hämin in höchstmöglicher Ausbeute erhalten wird.

Die Lösung dieser Aufgabe besteht in einem Verfahren zum Isolieren und Reinigen von Hämin aus Hämoglobin in salzsaurem Medium, wobei man es aus wäßriger Lösung oder Suspension von pH 0,5 bis 2,5 in Gegenwart von 0,005 bis 20 Gew.-%, bezogen auf die eingesetzte Lösung oder Suspension, eines kationischen, nichtionischen oder amphoteren Tensids oder einer Mischung derselben als Kristallisationskatalysator auskristallieren läßt.

In aller Regel wird Hämin aus Hämoglobin u.

dieses aus Blut gewonnen. Das in saurer Lösung bestehende Gleichgewicht der Hämoglobinspaltung in Hämin und Globin wird durch den Zusatz des Katalysators in Richtung auf das Hämin verschoben, weil dieses nun auskristallisiert (und damit der Rückreaktion entzogen wird). Es geht also im wesentlichen um die Kristallisation und Isolierung des Hämins aus Gemischen von Hämoglobin mit Globin.

Kocht man eine verdünnte, salzsaure Hämoglobinlösung, so scheidet sich bald ein dicker Niederschlag ab, der aus einem Gemisch von denaturiertem, also unlöslich gewordenem Globin und Hämoglobin besteht. Es wurde nun gefunden, daß bei dieser Behandlung durch Zusatz eines geeigneten Tensids als Katalysator keine Hämoglobinabscheidung erfolgt, sondern daß sich überraschenderweise Teichmann'sche Häminkristalle in praktisch quantitativer Ausbeute bilden. Sie setzen sich beim Stehen ab. Der farblose Überstand besteht aus einer reinen, salzsauren Globinlösung. Eine Verdünnung der Hämoglobinlösung auf 2 bis 8, insbesondere ca. 5% Trockengehalt vor der Hydrolyse wird zwar bevorzugt, ist aber nicht zwingend. Die Hydrolyse und Häminkristallisation läuft auch in konzentrierter Lösung, beispielsweise bei 15% Trockengehalt, ab, nur sind die Reaktionsgeschwindigkeiten größer, d.h. das Hämin scheidet sich in so kurzer Zeit ab, daß die Kristalle klein bleiben und sicht nicht oder sehr langsam absetsen. Andererseits ist das Arbeiten mit stärker verdünnten Lösungen weniger wirtschaftlich.

Als Katalysator eignen sich die genannten Tenside (also grenzflächenaktive Verbindungen mit hydrophilen und hydrophoben Gruppen), während anionische Tenside in anderer Weise reagieren. Von den zahlreichen marktgängigen Sorten lassen sich prinzipiell alle verwenden, bei den nichtionischen vorzugsweise die mit höherem, Molgewicht. Zur Erleichterung einer Trennung von Tensid und Globin (das am einfachsten nach der Abtrennung der Häminkristalle aus der Mutterlauge durch Neutralisieren gefällt wird) empfiehlt es sich, Tenside zu verwenden, die auch im neutralen pH-Bereich in Lösung bleiben.

Als Beispiele für geeignete kationische Tenside seien genannt: Quaternäre Ammoniumsalze, die einen oder 2 langkettige Alkylreste im Molekül tragen. Besonders wichtige Vertreter sind beispielsweise Dimethyl-$C_8$- bis $C_{18}$-Alkylbenzylammoniumchloride, wie Benzyldodecyldimethylammoniumchlorid, sodann Stearyltrimethylammoniumchlorid, Benzyltrimethylammoniumchlorid, Benzylmyristyldimethylammoniumchlorid. Cetylpyridiniumchlorid und Dihydroxyethylmethyloleylammoniumchlorid. Ferner sind zu nennen: Fettamine mit Oxy- oder Oxypolyäthergruppen wie Oktadecyldiäthanolamin oder Lauryldipolyglykolamin. Ferner Anlagerungsprodukte von Äthylenimin an die Salze von Fettaminen: 2-Fettalkylimidazoline und 2-Fettalkylbenzimidazole; Triäthanolamin-Fettsäuremonoester.

Als geeignete nichtionische Tenside seien beispielsweise genannt: Oxäthylierungsprodukte natürlicher oder synthetischer Fettalkohole ab 9 Kohlenstoffatomen. Speziell empfehlen sich in diesem Fall 9 bis 50-fach oxäthylierte und oxpropylierte Oxoalkohole, vor allem $C_9$- bis $C_{11}$-, $C_{12}$- bis $C_{15}$- und $C_{16}$- bis $C_{20}$-Schnitte als nichtionische Tenside. Ebenfalls sind Alkyphenole geeignet, insbesondere Octyl-, Nonyl- oder Dodecylphenol in Form ihrer 8 bis 50-fach oxäthylierten und/oder oxpropylierten Derivate. Kondensationsprodukte von Fettsäuren mit Alkanolaminen in 8 bis 50-fach oxäthylierter Form sind ebenfalls als Tenside geeignet. Geeignete nichtionische Tenside sind auch die bekannten Polymerisationsprodukte des Äthylenoxids und Propylenoxids mit Molgewichten zwischen 500 und 2 500, sowie Blockcopolymerisate aus Äthylenoxid und Propylenoxid mit Molgewichten zwischen 500 und 8 500.

Als amphotere Tenside sind die verschiedensten Betaine geeignet, sofern sie einen langkettigen hydrophoben Rest tragen, wie Myristylbetain oder die Gruppe der Sulfotaurine.

Die verschiedenen Tenside und Tensidtypen können auch gemischt eingesetzt werden. Die anwendbaren Tensid-Konzentrationen liegen in einem weiten Bereich, bei 0,005 bis 20, vorzugsweise 0,01 bis 5 Gew.-%, bezogen auf eingesetzte Lösung oder Suspension, mit Schwerpunkt bei 0,025%. Die Chloridionenkonzentration muß mindestens ausreichen, um das Hämin zu bilden; zweckmäßig ist sie (wesentlich) größer. Nach oben ist ihr keine definierte Grenze gesetzt.

Erhitzt man die Reaktionsmischung länger, als zur Kristallisation des Hämins erforderlich, so kommt es auch in Gegenwart der Tenside zur Abscheidung von denaturiertem Globin. Dieser Vorgang ist unerwünscht, weil sich dann ein Gemisch von unlöslichem Hämin mit Globin bildet. Für größere Ansätze, bei denen das Aufheizen und Abkühlen der Charge längere Zeit in Anspruch nimmt, wird deshalb die kontinuierliche Ansatzführung zwecks Kurzzeiterhitzung bevorzugt. Hierzu wird die mit Tensid als Katalysator versetzte Reaktionsmischung durch einen Wärmeaustauscher gepumpt, wobei sie rasch erhitzt wird, gegebenenfalls noch eine zeitlang bei der erreichten Reaktionstemperatur gehalten, indem ein Gefäß passenden Volumens durchströmt wird, und dann in einen zweiten Wärmeaustauscher zur raschen Abkühlung gebracht. Als Heizmittel kann jedes Medium dienen, wie heiße Wasser, Glycerin, Öl usw., aber vorzugsweise Dampf, der bei Normaldruck als Kondensat austritt, wodurch ein Kochen der wäßrigen Reaktionsmischung vermieden und doch eine Temperatur, die dicht unter ihrem Siedepunkt liegt, erreicht wird. Natürlich kann bei größeren Anlagen auch Dampf unter Druck verwendet werden, wenn eine entsprechende Temperaturregelung vorhanden ist. Bei 90—99°C erzielt man die günstigsten Resultate, während bei deutlich darunter liegenden Temperaturen die Globindenaturierung konkurriert. Durch eine der Reaktionsgeschwindigkeit angepaßte Pumpgeschwindigkeit kann der optimale Verlauf leicht eingeregelt werden, wie in den Beispielen 2 und 3 beschrieben.

Mit Hilfe von zugesetzten Hämin-Impfkristallen läßt sich nicht nur ein zeitlich präziser Reaktionsablauf gewährleisten, sondern auch eine beträchtliche (bis zu 99%) Einsparung an Tensid-Katalysator erreichen. Die Impfkristall-Suspension könnte prinzipiell auch ohne Tensidkatalysator die Häminkristallisation auslösen. Hierzu müßte die Kristallisationsgeschwindigkeit so gesteigert werden, daß sie noch vor der konkurrierenden Globindenaturierung beendet ist, d.h. es wäre eine entsprechend große Impfkristalloberfläche erforderlich. Man könnte z.B. eine große Menge an Impfkristallhämin einsetzen, d.h. etwa 10 bis 100 mal mehr als an Hämin entsteht, und besonders fein zerriebene Impfkristalle von unter 1 µm mittlerem Durchmesser verwenden, wobei das entstehende Hämin dann auch entsprechend fein ausfiele, so daß die zentrifugale Abtrennung schwierig wäre. Aufgrund dieser Problematik ist dieses (hier nicht beanspruchte) Verfahren dür die Praxis kaum von Bedeutung, obwohl es den Vorteil hat, daß kein Fremdstoff eingeführt wird, der das Hämin oder das Globin verunreinigen kann. Der erfindungsgemäß einzusetzende Tensid-Katalysator ist zwar ein solcher Fremsstoff, aber er ergibt so große Häminkristalle, daß diese sich spontan, d.h. ohne Zentrifugation, absetzen, und da die Tenside bei der Neutralisation der Globinhydrochloridlösung im Gegensatz zum Globin in Lösung bleiben, sind sie gut wieder entfernbar. Die Häminkristalle werden von Tensidresten durch Waschen mit Wasser befreit.

Der Einsatz von Tensidkatalysator ist ferner gut geeignet, um Rohhämin mit minimalem Substanzverlust umzukristallisieren. Unter Rohhämin wird nicht nur das in den Beispielen 2 bis 4 erhaltene Reaktionsprodukt verstanden, sondern auch jegliche anderen häminhaltigen Rückstände, wie sie beispielsweise beim enzymatischen Hämoglobinabbau entstenen (vgl. Beisp. 8) oder einfach unreines Hämin. Die Tensidkonzentration wird hier zweckmäßig höher gewählt, etwa im Bereich con 0,1 bis 20 Gew.-%, mit Schwerpunkt bei 5%, bezogen auf Lösung. Amorph ausgefallenes oder gelöstes Hämin kann direkt kristallisiert werden, während Kristallhämin zunächst in Ammoniakwasser gelöst werden muß. Anstelle von Ammoniak läßt sich auch eine beliebige andere Base verwenden, nur sind die Lüsungen oft unbeständiger. Man kann auch das Rohhämin in organischen Lösungsmitteln wie Dimethylformamid oder Dimethylsulfoxid lösen und dann mit so viel tensidhaltigem Wasser versetzen, bis die Mischung überwiegend Wasser als Lüsungsmittel enthält, und daraus das Hämin kristallisieren lassen.

Die in den Beispielen 5 bis 8 beschriebenen Reinigungskristallisationen führen zu Teichmann-Kristallen gezielter Größe. Besonders groß werden sie durch Anwendung von viel Tensid, wenn während der Kristallbildung nicht gerührt wird (Beispiel 7), besonders klein, wenn wenig nichtionisches Tensid unter Rühren (Biespiel 6) und rascher Salzsäurezugabe erhitzt wird. Impfkristalle sind hierbei nicht notwendig.

Wird eine ammoniakalische Häminlösung, die Tensidkatalysator enthält, rasch mit Salzsäure angesäuert, so entsteht ein Hämin-Niederschlag mit Kristallfehlern, z.B. gekrümmte Nadelreihen oder Knollen. Geht man aber von einer neutralen Lösung aus, z.B. in Dimethylformamid, so bilden sich normale Kristalle. Es zeigte sich, daß auch aus ammoniakalischer Lösung normale Kristallformen erhalten werden, wenn zunächst bei einem pH-Wert von 2 bis 5 erhitzt wird und erst danach Salzsäure langsam zugegeben wird, bis sich die ersten Kristalle bilden.

Bei rascher HCl-Zugabe werden die Kristalle kleiner und spitzer, was im Sinne einer glatten Isolierung meist nicht erwünscht ist. Deutlich ist auch ein Spezifischer Einfluß des jeweiligen Tenside aud die Kristallform erkennbar, die dann schlanker bis faseriger oder breiter bis lappiger, stärker überkreuzt bis sternförmig usw. sein kann.

Bei Rohhäminen, die mit Globin-Eiweiß verunreinigt sind, unterzieht man dieses ggf. einer enzymatischen Hydrolyse, z.B. mit Pepsin, um das Umkristallisieren und die Häminabtrennung zu erleichtern. Da durch mehrfaches Aufschlämmen mit Wasser und nachfolgendes Absetzenlassen on das Hämin oft nur mit einem Reinheitsgrad on 90% erhalten wird, kann noch eine Behandlung in 50 bis 70-%iger Schwefelsäure erfolgen, wie im Beispiel 5 beschrieben, wobei der Reinheitsgrad auf über 98% ansteigt.

Beispiel 1

Rinderblut wurde nach Zusatz von Trinatriumcitrat zentrifugiert und das verbliebene Blutkörperchenkonzentrat durch Verdünnen mit Wasser auf einen Trockengehalt von 5% gebracht. Man gab nun 20-%ige Salzsäure zu, bis der pH-Wert 1,5 betrug.

500 ml dieser Ausgangslösung wurden mit 10 g Benzyldodecyldimethylammoniumchlorid verrührt und auf dem Magnetrührer innerhalb von 10 Minuten zum Sieden erhitzt. Nach etwa 3 Minuten entfärbte sich die rotbraune Lösung unter Anscheidung schwarz-violetter Häminkristallchen. Man kühlte mit Wasser und ließ absitzen. Die überstehende, fast farblose Lösung von salzsaurem Globin ließ sich leicht abdekantieren. Der Bodensatz von Kristallhämin ergab nach dem Absaugen, Waschen und Trocknen 0,76 g Ausbeute = 100% der theoretisch möglichen Menge, mit einer Reinheit von 96,5%.

Beispiel 2

10 l von der im Beispiel 1 beschriebenen Ausgangslösung mit pH 1,5 wurden mit 24 g Cocamidopropyl-Betain (ein $C_7$ bis $C_{17}$ 1-Alkoylamido-3-dimethylammonio-propan-3-carboxymethylbetain) und mit 0,2 g einer 4-%igen Impfkristall-Suspension verrührt, die durch 6-stündige Feinvermahlung einer wäßrigen Häminkristallsuspension in einer Rührwerkskugelmühle (Tourenzahl ca. 400 U/min.) erhalten worden war (mittlerer Teilchendurchmesser bei 1 μm).

Diese Ansatzlösung wurde in kontinuierlicher Arbeitsweise von unten durch ein senkrechtes, ummanteltes, dampfbeheiztes Glasrohr von 1,40 m Länge und 20 mm Durchmesser gepumpt und anschließend über einen Laborkühler auf maximal 55°C abgekühlt. Die Fördergescheindigkeit wurde so eingestellt, daß die austretende Reaktionslösung nicht mehr braun gefärbt war, was unter den angegebenen Bedingungen etwa bei 80 ml pro Minute lag. Man trennte über eine kleine Durchlaufzentrifuge (ca. 3000 × g) und erhielt so 10 l leicht gelbliche Globinhydrochloridlösung und 170 g feuchtes, etwas globinhaltiges Roh-Hämin. Die weitere Aufarbeitung erfolgte wie im Beispiel 5 beschrieben.

Beispiel 3

600 l von der im Beispiel 1 beschriebenen Ausgangslösung wurden mit 150 g Nonylphenolpolyglykolether (Polymerisationsgrad 30) und 20 g der 4-%gen Hämin-Impfkristallsuspension von Beispiel 2 verrührt.

Diese Ansatzlösung wurde in kontinuierlicher Arbeitsweise von unten durch einen senkrecht stehenden, dampfbeheizten Röhren-Wärmtauscher von 1 m Länge und 46 Edelstahl-Röhren mit je 12 mm Durchmesser gepumpt und weiter über ein 6 l fassendes Rohrstück als Nachreaktor und einen Kuhler von ca. 0,1 m² Kühlfläche in einen kontinuierlich austragenden Separator mit 3 l Trommelgröße und etwa 5000 × g Schleudereffekt geleitet. Die Fördergeschwindigkeit der Pumpe betrug etwa 250 l pro Stunde und wurde so eingeregelt, daß die Lösung beim Eintritt in die Zentrifuge keine Braunfärbung mehr aufweis. Das Zentrifugat bestand aus etwa 600 l hellgeler Globinhydrochloridlösung; der Rückstand aus 4,2 kg feuchtem, globinhaltigem Roh-Hämin. Dessen Aufarbeitung erfolgte nach Beispiel 5.

Beispiel 4

Der Ansatz von Beispiel 3 wurde mit der 5-fachen Menge des gleichen Tenside = 0,75 kg und dafür mit nut 1/10 der Impfkristallmenge = 2 g Suspension wiederholt. Die Umsetzungsgeschwindigkeit war die gleiche wie im Biespiel 3, Es wurden 600 l salzsaurer Globinlösung und 4,4, kg feuchtes Roh-Hämin erhalten.

Beispiel 5

Das in den Beispielen 2 bis 4 erhaltene Roh-Häm in wurde durch Wasserzusatz rührfähig gemacht und 2 Stunden lang mit 1 g Pepsin pro 2 l Suspension bei einer Temperatur von 30 bis 40°C gerührt. Dann wurde mit Eis bis +5°C abgekühlt und das Hamin durch Zusatz von Ammoniak gerade vollständig in Lösung gebracht. Man gab nun, bezogen auf die zu erwartende Häminausbeute, einen gleichen Gewichtsteil Nonylphenolpolyglykolether (Polymerisationsgrad 30) und ebensoviel Citronensäure zu und erwärmte 10 Minuten land auf ca. 95°C. Dann versetzte man unter Rühren mit einer dem verwendeten Ammoniak äquivalenten Menge Salzsäure und gab bei 95°C langsam weiter Salzsäure zu, bis die Hämin-Kristallisation begann (Mikroskop). Nach ein bis zwei Stunden waren die amorphen, braunschwarzen Klümpchen vollständig verschwunden und dafür eine entsprechende Anzahl Teichmann'scher Häminkristalle von recht einheitlicher Größe mit ca. 0,02 mm Katenlänge entstanden.

Man ließ absitzen, dekantierte ab und versetzte den Rückstand unter Rühren mit 85-%iger Schwefelsäure, bis eine Konzentration von 60% an Schwefelsäure erreicht war. Nach 10 Minuten verdünnte man mit dem doppelten Volumen Wasser, ließ absitzen und wusch mit Wasser auf der Nutsche säurefrei. Die Ausbeute war quantitativ. Der Reinheitsgrad betrug 98%, gemessen als spez. Extinktion in NaOH bei 388 nm (1%, 1 cm) = 956 für 100-%iges Hämin.

Fe ber.: 8,57% gef.: 8,4%
Cl ber.: 5,44% gef.: 5.3%.

Beispiel 6

Das Umkristallisieren von Beispiel 5 wurde mit nut 1/10 der Katalysatormenge wiederholt. Es enstanden wesentlich kleinere Häminkristalle.

Beispiel 7

Das in Bsp. 5 beschriebene Umkristallisieren wurde mit der gleichen Gesamtmenge einer Katalysatormischung aus gleichen Teilen der Tenside von Beispiel 1 und 5 wiederholt und während der Kristallisation nicht gerührt. Es entstanden wesentlich größere Häminkristalle mit etwa 0,1 mm Kantenlänge.

Beispiel 8

40 l von der im Beispiel 1 beschriebenen Ausgangslösung wurden mit 10 g Pepsin 16 Stunden lang bei 37°C gerührt und dann über eine Durchlauf-Zentrifuge mit etwa 5000 × g Schleuderkraft und 3 l Trommelinhalt gegeben.

Der häminhaltige Rückstand betrug 2 kg und besaß einen Trockengehalt von 12%. Er wurde mit 2 l Wasser verdünnt, 60 g des Katalysators von Beispiel 3 zugegeben und auf 95°C erwärmt. Durch Zusatz von zunächst 150 ml, dann nach und nach jeweils 10 ml 20-%iger Salzsäure wurde die Kristallisation eingeleitet und weiter verfahren wie in den Beispielen 5 bis 7 beschrieben.

Ausbeute: 60 g Kristallhämin
Reinheit: 98%.

**Patentansprüche**

1. Verfahren zum Isolieren und Reinigen von Hämin aus Hämoglobin in salzsaurem Medium, dadurch gekennzeichnet, daß man es aus Wäßriger Lösung oder Suspension vom pH 0,5 bis 2,5 in Gegenwart von 0,005 bis 20 Gew.-%, bezogen auf die eingesetzte Lösung oder Suspension, eines kationischen, nichtionischen oder amphoteren Tensids oder einer Mischung derselben als Kristallisationskatalysator auskristallisieren läßt.

2. Verfahren nach Anspruch 1, dadurch gekennzeichnet, daß die Häminkristallisation kontinuierlich durchgeführt wird.

3. Verfahren nach Anspruch 1 oder 2, dadurch gekennzeichnet, daß die katalytische Kristallisation durch Zusatz von mikrokristallinen Impfkristallen unterstützt wird.

**Revendications**

1. Procédé d'isolation et purification d'haemine à partir d'hémoglobine, dans un milieu chlorhydrique, caractérisé par le fait qu'on la sépare par cristallisation d'une solution ou suspension aqueuse, d'un pH de 0,5 à 2,5, en présence de 0,005 à 30% en poids, rapportés à la solution ou suspension mise en oeuvre, d'un tenside cationique, non ionique ou amphotère, ou d'un mélange de ceux-ci, ent tant que catalyseur de cristallisation.

2. Procédé selon la revendication 1, cactérisé par le fait que la cristallisation de l'haemine est effectuée en continu.

3. Procédé selon la revendication 1 ou 2, caractérisé par le fait que la cristallisation catalytique est facilitée par l'addition de germes microcristallins.

**Claims**

1. A process for isolating pure hemin from hemoglobin in a medium containing hydrochloric acid, wherein the hemin is allowed to crystallize out from aqueous solution or suspension at pH 0.5—2.5 in the presence of from 0.005 to 20% by weight, based on the solution or suspension used, of a cationic, nonionic or amphoteric surfactant or of a mixture of these as crystallization catalyst.

2. A process as claimed in claim 1, wherein the crystallization of hemin is carried out continuously.

3. A process as claimed in claim 1 or 2, wherein the catalytic crystallization is augmented by the addition of microcrystalline seed crystals.